# EUROPEAN PATENT SPECIFICATION

(11) **EP 2 156 816 B1**
(45) Date of publication and mention of the grant of the patent: **18.09.2013**
(21) Application number: 08779154.7
(22) Date of filing: 07.05.2008
(51) Int. Cl.: A61H 39/08

(54) **METHOD OF ACUPUNCTURE AND A NEEDLE FOR CARRYING OUT SAID METHOD**
AKUPUNKTURVERFAHREN UND NADEL ZUR DURCHFÜHRUNG DIESES VERFAHRENS
PROCÉDÉ D'ACUPUNCTURE ET AIGUILLE POUR LE METTRE EN OEUVRE

(30) Priority: 07.05.2007 RU 2007117021
(43) Date of publication of application: 24.02.2010
(73) Proprietor: Frey Medical Technologies AG, 1003 Lausanne (CH)
(72) Inventor: MUKHINA, Mariat Muradalievna, Tver 170000 (RU); CHADAEV, Nikolay Veniaminovic, Tver 170000 (RU)
(74) Representative: Bugnion Genève
(86) International application number: PCT/RU2008/000285
(87) International publication number: WO 2008/136709

(56) References cited:
- EP-A2- 1 911 431
- WO-A1-98/35646
- JP-A- 08 098 708
- RU-C1- 2 286 133
- RU-C2- 2 289 391
- RU-U1- 26 402
- RU-U1- 26 402
- SU-A- 1 657 185
- SU-A1- 1 395 324
- SU-A1- 1 505 548

## Description

### Field of technology

The present invention relates to the field of medicine, and in particular to reflexotherapy and can be used to achieve a prolonged acupuncture action on a patient's body.

### Prior art

At the present time contemporary medicine is facing the problem of developing and optimizing those methods of treatment that exclude invasive (internal) administration of medicinal preparations and chemical substances. The topicality and urgency of this approach is connected with the increase in allergic reactions, the compromising of immune mechanisms, the development of dysbacterioses and drug-induced diseases in the human organism in response to an inappropriately frequent administration of pharmacological preparations, and also the deteriorating ecological situation on account of the increase in industrial pollution and contamination as a result of the intensive growth in industry. Such drug-free treatment methods include acupuncture, i.e. pricking biologically active points using a needle.

The scientific approach developed ancient Chinese medical technologies, which were already known 5,000 years ago. At the present time the development of treatment methods based on acupuncture is taking place in the direction of the discovery of new biologically active points and zones, used in acupuncture practice, and the choice of the most optimal regimes and methods of acupuncture to treat individual illnesses, and also in the direction of improving the technical basis used in needle reflexotherapy. One of these directions is the development of treatment methods using prolonged acupuncture action on a patient's body. In this connection, prolonged acupuncture action is normally understood to mean the action exerted by means of needles remaining in the tissues of a patient for a specific time after completion of a needle reflexotherapy session.

A method is known for prolonged acupuncture action, in which a prolonged acupuncture action is achieved by means of pricking a patient's ear at an acupuncture point with a perforated ring (US Patent 5465593, A44C 7/00 published 14.11.1995). In this method the action of the needle occurs only at one acupuncture point, which does not enable a sufficient intensive prolonged action to be achieved, and at the same time its intensity decreases over time on account of the occurrence of an adaptation effect. Furthermore, the proposed method has a limited sphere of application.

This document further describes an acupuncture needle, comprising a body and at least one movable fixing means made of a generally circular disc and provided with a through aperture, said fixing means being installed on the body of the needle and capable of sliding, and its securement in a given position is effectable by a pair of spring members provided on opposite sides of the fixing means.

EP patent 1911431 discloses an acupuncture needle comprising at least one fixing means, installed on the body of the needle, and its securement in the given position is effectable by bending one end of the body of the needle.

The closest analogue to the described method, is a method for achieving a prolonged action at acupuncture points, comprising determining acupuncture loci (needle prick points), one of which is selected as needle entry point and another of which is selected as needle exit point, following which these points are pricked by piercing them with a needle fixed by means of a locking means mounted on the end of the needle at the site of the needle exit on the surface of the skin (RU Patent 2289391, A61H 39/00 published 20.12.2006). Such a method enables a treatment channel to be created between two acupuncture points, whereby needles can be kept in the channel for a long time (a year or more).

The aforementioned method, which enables the intensity of the prolonged action to be increased and partially overcomes the adaptation effect, has found wide use in medical practice to treat obesity, in which connection the treatment channel was formed by pricking the two points AT17 and AT18 with one needle on the earlobe in the region of the tragus, fixing the needle in this position, and leaving it in the earlobe for several months or more. Some 12,000 patients have been treated by the aforedescribed method over three years, a positive result being obtained in more than 80% of patients. At the same time, in about 15-20% of patients the treatment results proved to be worse than expected on account of the fact that the needle became detached. Also, it is necessary to adopt additional measures in order to ensure an optimum adjustment of the strength of the acupuncture action and overcome the adaptation effect on account of its negative influence on the efficacy of treatment using prolonged acupuncture action. Besides this, the use of the above method in the treatment of some rhinological conditions can cause problems in relation to the choice of an appropriate procedure, since the recommend acupuncture points can be fairly remote from one another, which creates an obstacle for the action of "threading with one needle" and, consequently, limits the available choice of points.

### Essence of the invention

The technical object that is solved by the described method is the creation of an acupuncture method with prolonged action, enabling the curative effect to be improved by means of overcoming the adaptation effect, changing the intensity of the reflexogenic action and preventing complications, and also broadens the choice of methods that can be used in the treatment of various rhinological conditions.

The aforementioned objects are achieved in that the known acupuncture method with prolonged action, in which the acupuncture points or loci are determined, the needle entry and exit points are selected, the needle is passed through the selected entry and exit points, both ends of the needle remain outside the patient's skin, the needle is secured in this position by means of locking means mounted on the needle ends, and after finishing the acupuncture section the needle is left in the patient's body for a specified time, the new features is that acusal tracts are formed in different histological layers of the patient's skin by introducing needles, and as entry and/or exit points of each needle points are selected that are located in the region of the acupuncture point and/or reflexogenic zone and/or indifferent zone.

In addition the histological layer is chosen from the group comprising: epidermal layer, epidermis membrane, papillary layer, reticular layer, epichondral layer, chondral layer, subcutaneous-adipose layer, muscle layer, ligament, fascia or tendon.

Furthermore, when carrying out an acupuncture session the needles are installed so as to form acusal tracts only in one of the histological layers of the patient's skin, and the formation of acusal tracts in other layers of the skin is performed by inserting needles in subsequent acupuncture sessions.

Furthermore, when conducting an acupuncture session needles are installed in order to form acusal tracts in various histological layers of the patient's skin.

Furthermore, when conducting an acupuncture session needles are installed to form acusal tracts in one or several histological layers of the patient's skin after removing the needles installed during the previous session.

Furthermore, when conducting an acupuncture session needles are installed to form acusal tracts in one or several histological layers of a patient's skin without removing the needles that were installed during a previous session.

To achieve these objects an acupuncture needle according to claim 1 is proposed.

When fixing the needles *in situ* there is used at least one movable locking means installed on the body of the needle with the possibility of sliding, and its securement in the given position is effected by plastic deformation of the body of the needle.

Furthermore, a needle is provided with locking means mounted on the ends of the needle, wherein at least one of the fixing devices is movable and is installed so as to be able to slide along the body of the needle.

Furthermore, the body of the needle is made rigid in the form of a rod, or is flexible in the form of a cord or string.

Furthermore, the body of the needle is made of a plastics-metal alloy.

The formation, by means of installing needles, of acusal tracts in various histological layers of the patient's skin enables the intensity of the reflexogenic action to be altered, so as to match physiological processes occurring in the histological environment of the acusal tracts, which on the one hand enables the adaptation effect to be overcome, and on the other hand enables possible complications to be avoided.

The choice, as entry and/or exit points of each needle, of points located in the region of the acupuncture point, and/or reflexogenic zone, and/or indifferent zone, enables the intensity of the reflexogenic action to be altered and broadens the choice of treatment.

The choice of histological layer from the group including: epidermal layer, epidermis membrane, papillary, reticular, epichondrial, chondrial, subcutaneous-adipose, muscle layer, ligament, fascia or tendon enables the adaptation effect to be overcome by a change in the intensity of the reflexogenic action and enables possible complications to be overcome.

The installation of a needle for forming acusal tracts only in one of the histological layers of the patient's skin and the formation of acusal tracts in other layers of the skin by introducing needles at subsequent acupuncture sessions enables the intensity of the reflexogenic action to be more accurately regulated and prevents possible complications.

The installation of a needle for forming acusal tracts in various histological layers of a patient's skin enables the intensity of the reflexogenic action to be increased and broadens the choice of treatment.

The installation of needles for forming acusal tracts in one or several histological layers of the patient's skin, implemented without withdrawing needles installed during a previous session, enables the intensity of the reflexogenic action to be increased.

The use, for fixing a needle, of at least one movable fixing means installed on the body of the needle so as to be able to slide, enables a reliable securement of the needle on the patient's body to be effected at the time of treatment, which is one of the necessary conditions ensuring efficacy of treatment and prevention of possible complications.

The formation of the body of the needle either as rigid in the form of a rod, or flexible in the form of a string or cord, enables the choice of procedure or a given method to be broadened.

The formation of the body of the needle of a plastic-metal alloy ensures the reliable securement of the fixing means in the specified position by means of plastic deformation of the body of the needle.

The essence of the invention is explained with the aid of the following drawings, in which:
Fig. 1 is a general view of a needle;
Fig. 2 is a view of section I in Fig. 1 (enlarged).

### Preferred embodiments of the invention

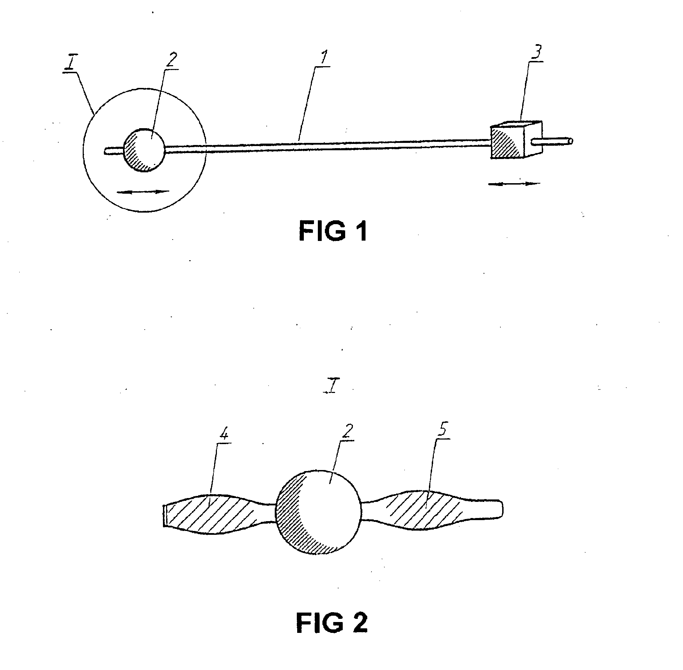

In order to realize the described method a needle 1, shown in Fig. 1, may be used, the body of which is designed to be rigid in the form of a rod or flexible in the form of a cord. Two fixing means 2 and 3 are installed on the rod or cord of the needle 1, arranged on opposite ends of the section of the needle, wherein one of the fixing means or both fixing means are designed to be movable and are installed so as to be able to slide along the body of the needle. The securement of the movable fixing means in the specified position is effected by means of plastic deformation of the end sections of the needle remaining outside the skin. In order reliably to secure the fixing means it is sufficient to carry out a plastic deformation of the body of the needle by compressing it to a deformed state (flattening), for example with the aid of forceps, which is effected from the outside (i.e. not facing towards the patient's body) of the fixing means. After this, the width of the needle at the site of compression will exceed the diameter of the through aperture of the movable fixing means, which ensures its reliable locking action. If necessary, the fixing means may additionally be secured to the body of the needle by flattening it from the inside (i.e. the side in contact with the patient's body) of the fixing means (Fig. 2). The needle can be made of malleable metals and alloys known in needle reflexology, that do not produce an allergic reaction on contact with metal, for example of an alloy consisting of 75% gold, 13% silver and 12% copper (see for example RU Patent 26402 A61B 17/06, published 10.12.2002). The needles may be of various sizes, determined by the choice of insertion site and/or the action parameters.

In order to carry out acupuncture the acupuncture points are determined in accordance with a procedure recommended for treating the condition from which the patient is suffering, wherein the entry and exit points of each needle are chosen from points located in the region of the acupuncture point and/or reflexogenic zone and/or indifferent zone. The needle 1 is then inserted through the selected entry and exit points, forming an acusal tract in the corresponding histological layer of the patient's skin, and both ends of the needle remain outside the patient's body. Following this the needle 1 is secured in this position by means of fixing means 2 and 3 mounted on the ends of the needle, at least one of said fixing means being movable. The movable fixing means are locked in position by compressing the corresponding external section of the body of the needle (beyond the limits of the fixing means) so as to achieve a deformed state (flattening). The free ends of the needle projecting beyond the limits of the fixing means are cut off with the aid of forceps. On completion of the acupuncture session the needle is left in the patient's body for a specified time, which may be one year or more. Depending on the chosen treatment procedure several needles may be employed, each of which is inserted in a different histological layer. However, this does not exclude a variant in which one needle can be installed directly in several histological layers. As a rule, when carrying out an acupuncture session the needles are installed to form acusal tracts only in one of the histological layers of the patient's skin, and the formation of acusal tracts in other layers is carried out by inserting needles during subsequent acupuncture sessions. In this connection, depending on the required treatment dynamics, the insertion of needles during subsequent acupuncture sessions can be carried out with the removal of needles inserted during a previous acupuncture session, as well as with the composition of previously inserted needles, which normally enables the reflexogenic action to be intensified.

Various alternatives may be employed when choosing points located in the region of an acupuncture point and/or reflexogenic zone and/or indifferent zone, as the entry and/or exit points of each needle:
- from point to point;
- from a point in the reflexogenic zone;
- from a point in the indifferent zone (beyond the limits of the reflexogenic zone);
- from a reflexogenic zone to point;
- from a reflexogenic zone to a reflexogenic zone;
- from a reflexogenic zone to an indifferent zone;
- from an indifferent zone to a point;
- from an indifferent zone to a reflexogenic zone.

In this connection, the "from point to point" and "from reflexogenic zone to reflexogenic zone" variants include, *inter alia,* cases where the entry and exit points of a needle may be located respectively within the limits of the boundary of one point or one zone.

Increasing the range of choice of entry and exit points of a needle broadens the scope of formation of an acusal tract and, consequently, the possibility of a method as regards the choice of procedure to cure various rhinological conditions, and also enables a more selective action to be achieved and overcomes the adaptation effect in a prolonged action process.

In this connection the "reflexogenic zone" concept known from the relevant art, for example from the monograph "Basic mechanisms of acupuncture treatment", N.A. Nikolaev, Riga, 1998, is used in the description of the described method, according to which:
- the term reflex is understood to mean a rapid response of various biological structures to a defined, specific and localized stimulus arising in addition to pain control in a human (as on page 6), and the main factor is the peripheral excitation of any reflex;
- the term reflexogenic zone is understood to mean a peripheral zone of action that forms the start or end of the corresponding reflex ark, creating either a diagnostic zone, or a site of application of a curative action. Doctors know from semiotics points that are characteristic of certain conditions and diseases, and Head A zones are also well known to doctors (as on page 15).

At the same time an acupuncture point can be regarded as a partial case of a reflexogenic zone. A reflex peripheral zone is point-like for body acupuncture points and auriculotherapy points. As like the Weihe point, used in homeopathy, the majority of known acupuncture points are already larger in terms of diameter (within thumb-size limits). The Hannemann A point (also used in homeopathy) has a,similar size. The Head a zones are substantially broader - their size may be up to several centimeters. This is also true of Jarricot dermalgic zones, which normally have a round or oval contour (Jarricot H., 1932 - Sur certains etats douloureux: visceralgies, dermalgies reflexes, cellules et quelques phenomenes reflexes d origine therapeutique Essai clinique et therapeutique. Medical thesis, Lyon).

When treating patients using the described method, the work is basically carried out on the dermalgic zones (pain zones on the skin, connected with the internal organs and systems).

As a histological layer there is chosen a layer from the group comprising: epidermal layer, epidermis membrane, papillary, reticular, epichondrial, chondrial, subcutaneous-adipose, muscle layer, ligament, fascia or tendon.

In this connection, it should be noted that the "channel" formed according to the described method does not relate to an acupuncture channel or meridian in the conventional meaning of the term meridian used in reflexotherapy. This "channel", in contrast to normal acupuncture channels that connect, by an imaginary conducting but non-material line, energy flows between strictly defined points, mechanically connects any separately taken acupuncture points according to the procedure prescribed by the doctor, and a material channel is physically formed at the location site of the needle as a result of epithelization processes. In this connection, in order to emphasise the difference between the aforementioned channel and the conventional concept of an "acupuncture channel" in the present description the term proposed by the author, namely "acusal tract" (from the Latin aqueous - needle) will be used to denote this channel.

The prolonged action mechanism of such an "acusal tract" treatment channel may be explained as follows. As is known, when needles are inserted for a long time (prolonged action) there is adaptation to the reflexogenic action, which reduces the efficacy of the treatment and increases the recovery time. The formation of an "acusal tract" by the described method ensures that the necessary intensity of the reflexogenic action is maintained in the tract for a sufficiently prolonged time, thereby enabling inflammatory complications to be avoided. In this connection, the intensity of the reflexogenic action in the tract has a tendency to increase in a cascade-like manner, since it correlates in a gradient manner with physiological processes occurring in the histological environment at the needle location site. As is known, an initial stream of impulses is formed directly at the moment of insertion of the needle. Then, after passing the needle through the selected points (threading points), secondary streams of impulses are formed, which are more powerful, permanent and prolonged. These streams of secondary impulses from the reflexogenic zone are caused by physiological processes occurring in the tissues. In this connection, the three stages of processes occurring in the skin may be distinguished, namely impulses arising at each stage, sub-divided into three types:

### 1^{st} Stage - epithelization of an acusal tract. Up to 30 days.

As a result of the formation of an acusal tract, an acusal tract space with two openings or apertures, formed by the needle, is created. According to the regeneration laws, the tissue around the needle starts to undergo epithelization, forming an epithelized channel. In the process of epithelial enlargement during the course of the gradual and prolonged epithelization by receptors of the cells, a powerful stream of secondary microimpulses of the first type flowing from the reflexogenic zone to the subcortical centres of the hypothalamus-hypophysis-physis system is generated, which intensifies the curative effect of the given action. In particular, when treating obesity an inhibition focus is created fairly rapidly in the hunger centre in the hypothalamus and lipolytic mechanisms are activated, and as a consequence there is a fairly intensive loss of body mass. When treating arterial hypertension the afferent impulses potentiate the angiosuppressant effect in the vasculomotor centre in the hypothalamus, and the arterial pressure is normalized fairly rapidly. In the case of the treatment of an allergy the activity of the antihistamine mechanisms is intensified, which alleviate the allergic reaction fairly rapidly. For patients with a more complicated medical history and a fairly pronounced illness or medical condition, which requires a more prolonged action lasting from one to several months, the important therapeutic moment is the action by the secondary impulses of the second type, which are produced in the 2^{nd} stage.

### 2^{nd} Stage - regeneration of an acupuncture channel.

When epithelization of an acusal tract has finished, the surface of the formed channel will be covered by a multilayered dense keratinizing epithelium, the epidermal-outer layer of the cells of which gradually desquamates. Renewal of the epidermis takes place due to its deep-lying growth layer ("human anatomy", Borzyak E.I. et al., Vol. 2, Moscow, Meditsina, 1987, p. 469). This regeneration process of the cells of the epidermis is a powerful stimulus of exteroreceptors, converting the stimulation energy into nerve impulses, directing a powerful stream of neurogenic afferent impulsation ("human anatomy", Borzyak E.I. et al., Vol. 2, Moscow, Meditsina, 1987, p. 290), to nuclei in the brain. According to the reflex theory, which is generally accepted in medicine - the larger the pulses from the periphery, the more pronounced are the processes in the central structures, namely ganglionic nodes and subcortical centres of the brain. Thus, the secondary impulsation of the second type intensifies the curative effect.

### 3^{rd} Stage - Intrachannel extumation (from the Latin extumio - to swell).

When a needle is left in an acusal tract for four months or more an accumulation of cells that have pealed off from the epidermis, neutrophils and macrophages, and dead bacteria accumulates in the lumen of the channel. In practice, on removing the needle at this stage a whitish coating can be seen on the needle, and sometimes this coating in the form of a rod can be removed by pressing a swab onto the outer wall of the acusal tract. Over time there will be a gradual accumulation of the aforementioned metabolic products of the microorganisms and from the accumulation of the metabolic products of the tissue structures in the acusal tract a pressure is exerted on its internal walls with the effect of dilating them, and the longer the needle is left in the channel the more the accumulated contents of the channel will press on its walls and, consequently, the more pronounced will be the passive pressure impulsation. These impulses are classified as secondary impulses of the third type. They enable the maintenance of the dominant bond in the previous stages, and also potentiate the curative effect. In this connection, since the acusal tract is an open tract and permits the discharge of exudate and outflow of used organelles, cells and other metabolic products of microorganisms on the surface of the skin, there will be no superfluous decay processes in the channel and, accordingly, no formation of conditions for the development of inflammatory complications.

However, when a needle is left *in situ* and an acusal tract is formed in one of the layers of the skin, after a certain time, which is individual for each patient, the receptors in the acusal tract will again become habituated, i.e. adapted to the number and strength of the impulses. In order to overcome this effect it is necessary to intensify the flow of impulses either afterwards or at the same time. For this, it is proposed to form acusal tracts in different histological layers of the patient's skin, for example passing in turn through the epidermal layer of the skin, papillary layer of the skin, reticular layer of the skin, and subcutaneous-adipose tissue. The choice of the sequence of histological layers of the patient's skin for forming acusal tracts is determined on the basis of the particular features of the patient's organism and the nature of his/her illness, and also taking into account the specific features of each histological layer from the point of view of its suitability for the purposes of prolonged acupuncture reflexotherapy. At the same time each histological layer has its own structural features, innervation and blood circulation, and also its own cellular composition. The use of these special features also enables the new technical result to be obtained.

It is recommended to start insertion of the needles from the epidermal layer of the skin. Then, after determining the entry and exit points of the needle, located in the region of the acupuncture point and/or reflexogenic zone and/or indifferent zone, the needle is inserted into the epidermal layer of the skin, emerges at the surface and is fixed *in situ,* and is then left in this position for a specified period of time depending on the nosology. Then, in the subsequent session, the needle is removed or left in the same place if it is necessary to intensify the action. One criterion for the location of a needle in a given layer of skin is the translucence of the rod of the needle underneath the epidermal cells. The result of inserting a needle in the given layer will be the formation of a more gentle and finer impulsation for a careful start of the treatment, and also the possibility of carrying out a trial treatment so as not to provoke, on account of too powerful an impulsation, any exacerbation of the illness or any other complications arising from the acupuncture treatment, in other words to check the reaction of the organism to acupuncture treatment. The need for such an approach is due to the problem of the existence of an individual intolerance to acupuncture in some patients, which all manuals on acupuncture warn about. If such a reaction occurs after inserting needles, the patient comes out in a cold sweat, becomes pale and loses consciousness. The formation of a powerful stream of afferent impulses lies at the basis of the etiological mechanism of such a pathological reaction, and arises in the acupuncture zone under the action of the needle. Impulses are directed by the afferent stream to the hypothalamus-hypophysis zone, to vegetative centres located there, which cannot handle this information with the result that a malfunction occurs, manifested in the aforedescribed symptoms. In this case it is basically recommended to remove the needle. In other words, in such a case it is absolutely clear that acupuncture therapy is contraindicated if some symptoms occur and the patient is not suited to such an effective method of treatment as acupuncture. The action itself in the epidermal layer solves the problem of preparing the organism and vegetative centres for the stream of afferent impulses and preventing possible pathological reactions to acupuncture. Since the impulses will be minimal when a needle is inserted into a given layer of the skin, then unpleasant sensations during this will in practice be neutralized, in other words this can be compared with a "setback" that a person receives in the course of daily life, which he/she did not even notice. Thus, the occurrence of the initial action in the epidermal layer provides a gentle initiation to the treatment and is a preventive measure for avoiding a pathological reaction of the body to acupuncture, which enables the individual susceptibility of patients to the acupuncture action to be overcome. The needle is then inserted into the epidermal membrane, its end exiting on the surface of the skin, where it is fixed *in situ* by a locking means. Then there is also the exposure time, which in the case of prolonged action can be up to 60 days or more. In practice the development of an adaptation is observed within 60 days.

In order to prevent adaptation, intensify the effect and renew the impulsation, in the following session the needles are inserted in the patient using the same method, but in the papillary layer of the dermis. The papillary layer is characterized by a porosity, and accordingly when inserted into this layer the needle will be somewhat translucent, and the doctor's hand will when installing the needle appear as it were in the unformed connective tissue.

The result of the formation of an acusal tract in the given layer will be an activation of impulsation due to the involvement of new receptors, and also due to biochemical processes occurring on resorption of microdiapedetic hematomas formed on traumatisation when introducing a needle into vessels of small diameter, a large number of which are characteristic of the given layer of skin. The installation of a needle in the patient is carried both when removing the previous needle, and when leaving it in the action zone. The following session is also decided according to the relevant indications.

In the following session the acupuncture procedure is repeated according to the same protocol, though the action takes place in the reticular layer of the dermis. The needle is left in place for the necessary time for exposure. The reticular layer, in contrast to the more superficial papillary layer, situated immediately underneath the epidermis, consists of compact unformed connective tissue containing a large number of collagen and elastin fibres and a small number of reticular fibres, and is the most compact of all the layers. Information on the structure of this dermal layer is given in known handbooks ("Human Anatomy", Borzyak E. I. et al, Vol.2, Moscow, Meditsina, 1987, p.470). The criterion for introducing a needle into this layer will be the sensation of a compact tissue environment and the attendant need to force the needle with a certain powerful thrust and pressure into the tissue. The result of the action of the needle in the given layer will be a renewal of the nature of the impulsation due to the involvement of new receptors and, as a consequence, the prevention of adaptation.

In some cases, in order to improve the reliability of the fixture of the needle it is recommended to insert it in the sub-reticular layer since a layer of elastin and collagen fibres will assist the formation of a unique corset, which will prevent exit of the needle through the skin. The criterion for introducing a needle into this layer will be the sensation of a hand passing with the least resistance into the medium. A better fusion of the needle under the reticular layer is also due to the fact that this needle, located under this layer, will in fact lie in the subcutaneous tissue containing fat globules. In fact, this is permeated by a deep dermal arterial network, i.e. a good blood supply is ensured. This means the optimal occurrence of all repair processes, and also a good supply of nutrients for the formation of the aforementioned tract (channel). The deep lymphatic network is also in fact formed at the boundary with the subcutaneous-adipose tissue, and lymphatic vessels of the deep network are connected to vessels of the fascias, muscles, and are directed to regional lymph nodes. Accordingly, a good lymph drainage and the absence of decay phenomena in this zone will also facilitate regeneration of the cells for the renewal and epithelization of the acusal tract. The reliability and strength of the walls of the tract that is formed for the prolonged action at the points is due to the above anatomical features, and also means that a needle will be reliably retained in the tract. It should also be noted that the anatomical feature of the sub-reticular layer consists in a highly expressed plexus of nerve fibres of somatic, sensitive (praneal, cerebrospinal) systems and nerves of the vegetative systems specifically in the sub-reticular layer and upper layers of the subcutaneous-adipose tissue. It is known that with a good innovation most impulses pass along the afferent fibres to the subcortical centres of the brain. The more a stream of impulses is created, the more expressed is the therapeutic effect from the given action.

The layer of subcutaneous-adipose tissues will be the following histological layer. This is particularly important for use in listing corporal points. Exactly the same result will be boosted in the given case on account of the features of the structure and blood supply of the fatty cells, the traumatisation of which will increase the choice of biologically active substances (histamines, prostaglandins), as a result of which the stimulation of the receptors will be even more intense, and the therapeutic effect will be more powerful. The stream of impulses formed in the acusal tract situated in the subcutaneous-adipose tissue, potentiates the curative effect of the whole acupuncture session.

In certain clinical cases it is recommended to carry out the aforementioned procedure in the perichondrial layer. This is a cartilage lining, for example on the auricle. A particular feature of this layer is the presence of collagen fibres arranged in one plane in the form of parallel fascicles. An acusal tract can also be formed in this layer for one year or more. A result will also be the involvement of new receptors, and due to this the renewal of impulsation and prevention of adaptation to the acupuncture action. Bearing in mind the need in the case of some nosologies to interact at points located on the auricle, the implementation of the above method can also take place in cartilage tissue. Isogenic cellular groups of chondrocytes, surrounded by an intercellular homogeneous substance consisting of protein glycanes form the basis of the above histological medium.

The creation of an acusal tract in the muscle layer is important in terms of listing. A particular feature of a muscle layer is the good blood supply and innovation. Muscle tissue is characterised by an involuntary contraction of the muscle fibres, which have ramifications that combine and unite, thanks to which a branched muscle network is formed. Accordingly the distribution of impulses is propagated over the whole receptor zone of the muscle, which involves new receptors, intensifies the impulsation, and prevents the development of adaptation. Thanks to these particular features renewal of impulsation will take place due to the interconnection of new receptors, and consequently prevention of adaptation to the acupuncture action.

The connection of the receptor apparatus of the ligaments is the next variant of implementation of the said method. The compact fibrous unformed tissue of ligaments, fascias and tendons consist of fibroblasts, elastin and reticular fibres.

In practice cases occur where, after exerting an action on all the aforementioned layers, it is necessary to additionally intensify the impulsation and renew the nature of the impulses, involving new receptors. For this, the creation of a curved tract was employed, one branch of which passes through all the histological layers up to the tendons, and another branch of which exited through these same layers, as if it had made a deflection in the ligaments. This measure enabled the effect of the procedure to be intensified, as a result of which a rapid therapeutic result was achieved.

The given order of the layers for forming acusal tracts is recommended and does not restrict the choice of possible variants of use of the described method, since in practice the specified choice, as mentioned above, it determined on the basis of the specific situation and details of each histological layer. Also, the described method enables a plurality of needles to be inserted immediately in the selected histological layer in one acupuncture session.

The results of the use of the described method can be illustrated by the following examples from practice.

Example A. A patient presented complaining irregular periods and pain in the region of the small pelvis during her periods. The patient had suffered from ovarian dysfunction for three years. Treatment with drugs was contraindicated on account of an allergy to medicinal preparations. Conventional acupuncture did not produce a permanent result.

It was decided to carry out a course of prolonged acupuncture therapy with the formation of an acusal tract in various histological layers. Before the acupuncture session a selection was made, with the aid of electrical point diagnosis, of a number of the most representative points on the auricle: AT55 (shen-men), AT58 (womb), AT82 (diaphragm), At83 (middle of the ear). Having defined the topography of the points, a needle was inserted at point AT55 into the superficial epithelial layer, and exited from point AT58, following which the needle was secured in the said position with the aid of a fixing means. The same procedure was performed with another pair of acupuncture points. A needle was inserted through AT82 into the epithelial layer, passed through this layer and exited from AT83. This action enables a fine stream of impulses to be generated for an input action on the subcortical hypothalamic structures. The needles were left in place for 1.5 months. In the following session the needles were removed, and the action was effected through these same points, but now in the papillary layer of the dermis and the needles were left in place for a further two months. The papillary layer contains a large number of small calibre vessels forming a capillary network. Diapedetic hematomas were formed by the action of the needle, and during their resorption there was a further hyperproduction of impulses on account of this fact. The sensation of a needle passing through a soft substance can be used as a criterion for placing the needle in this layer. The patient noted an improvement in her overall well-being during her periods, and a decreased pain level. After removing the needles from the papillary layer the needles were inserted by the aforedescribed method of prolonged action into the reticular layer of the dermis, which is a more compact and denser layer of skin and contains elastin and collagen fibres and a large number of receptors. Regeneration of the action on the new tissue layer intensifies the impulsation and prevents development of an adaptation. If it is necessary to improve retention of a needle, then it is best to insert the needle underneath this layer since this corset of collagen and elastin fibres will hold the needle in place and prevent it becoming detached. The needles were left in place for three months. At the next session the patient reported a marked improvement in her general well-being, although the duration of her periods still remained high, of the order of three days. Following this it was decided to form a curved acusal tract, going through all the layers. The needles were left in the same receptor points for a further three months. After the same course of treatment using the new method of prolonged action the patient noted an improvement not only in her overall well-being, but also the disappearance of all pathological symptoms - her periods were no longer painful and were regular. Following this. no indications for continuing action were noted and the needles were removed from the patient's auricle.

### Example 1. From a point to a reflexogenic zone

A patient, age 48, presented with exacerbation of trigeminal neuralgia. The patient complained of irradiating acute pain like lumbago in the region of the auricle and inner ear. Patient's medical history: the patient had suffered from trigeminal neuralgia for 10 years. The attacks were provoked by excessive cold and nervous stress. The trigeminal neuralgia was exacerbated as a rule every 1-2 months. Before an attack the patient noted that she had been in a cold place for a long time. The painful attacks occurred during the day, but were particularly severe at night, and were scarcely alleviated by pain-relief preparations; the patient could not sleep and was unable to work during the day. Objective findings: on palpation of the auricular zone a sharp painful sensation was noted at point IG19 (tin-gun), with irradiation of the pain into the interior of the ear, and also along the zygomatic arch to the region of the orbit (eye socket).

In order to alleviate the above symptoms it was necessary to carry out a long sedation of this point by the prolonged action method. For this, acupuncture was carried out at the point, the end of the needle exiting on the outside of the skin within the given acupuncture point, but in line with the existing innovation zone of the maxillary zone (second branch of the trigeminal nerve). The needle was inserted into the papillary layer of the dermis and fixed in position outside the skin by means of removable fixing means mounted on both sides of the needle. The next appointment was three weeks later. At this appointment the patient did not complain of any painful symptoms. In her words, there were no painful attacks during the night, she slept well, and the intensity and frequency of the attacks reduced immediately the following day after the treatment session, and in three days the painful attacks had completely disappeared. In order to consolidate the result, bearing in mind the chronic nature and duration of the periods of exacerbation of the neuralgia, the needle was left in place for a further 1.5 months. After 1.5 months the pain had not returned and there were no indications for leaving the needle in place. The needle was removed from the acusal tract.

### Example 2. From a point to an indifferent zone

The needle was inserted at one point, and exited at any suitable part of the skin.

Patient age 7. At the session the father said that the child was suffering from involuntary nocturnal incontinence. The child had suffered from this condition since birth. The child was treated with drugs by a neuropathologist, but with no result. Conventional acupuncture was carried out with repeated courses, but without any positive outcome. The frequency of night time incontinence was sometimes two to three times a night. There were no objective-pathological visible defects of the urinary system, and the urinalysis was normal. Sphincter-detrusor asynergy was diagnosed.

In order to treat the enuresis the child was prescribed prolonged action acupuncture selectively at the point RP6 (san-intsyo) from both sides on the internal surface of the lower third of the tibia. For this purpose the needle was inserted at the point RP6, and the lower end of the needle was inserted 2 cm higher at a suitable site, and fixing means were slipped on at both ends. The bandage was removed after a week. The acusal tract formed in the subcutaneous-adipose tissue had epithelized, the skin was clean, the needle moved freely in the channel, and was prevented from falling out of the channel by fixing means at both ends. After 10 days' prolonged treatment the involuntary enuresis no longer occurred every day, and after three weeks there were clear intervals of 3-4 days between the enuresis. In the following two months the frequency of enuresis was only once a week and the attack became much less frequent in the following three months, and did not recur. After six months the needles were removed from the acusal tracts.

### Example 3. From an indifferent zone to a point

Patient age 18 presented complaining of sweaty hands. This symptom had appeared during puberty when he was 14 and became worse as he got older. The symptoms became worse when the patient was agitated and nervous, which exacerbated the state of psycho-emotional stress, resulting in the patient feeling extremely uncomfortable. Furthermore, the patient is learning to drive, and the steering wheel, sliding in his wet palms, is a threat to both his life and that of pedestrians. Among the methods used to treat the condition, a neuropathologist prescribed drugs for six months, and also periodic courses of acupuncture reflexotherapy according to conventional methods for one year. No significant therapeutic results were achieved. Objective findings: patient's condition satisfactory. Skin of normal colour. On examination a pronounced hyperhydrosis of the palms and axial regions. On investigation no abnormal pathological signs in the internal organs were detected.

For the treatment, the most well-known general action point was selected, recommended for treating, in particular, vegeto-vascular dystonias - xe-gy (GI4). In order to create the maximum powerful stream of impulses needles were introduced from the zone adjacent to the above acupuncture point and passed through various histological layers, namely epidermal, papillary, reticular, subcutaneous-adipose network, and muscle layer, and then exited at the same point on the surface of the skin and were fixed in position by locking means. Thus, five acusal tracts were simultaneously formed, from which a powerful stream of impulses was directed along the afferent neurons to the hypothalamus-hypophysis zone. There an afferent synthesis occurred, the pulses returning along efferent neurons to the sweat glands located in the reticular layer of the skin of the palm of the hand and axillary (sub-muscular) region. This signal balanced and normalised the dysfunction of the sweat glands, and in particular excessive production of sweat by the cells of the sweat glands. As a result, a therapeutic result was achieved more rapidly than in the case of conventional acupuncture, and in fact within two months the sweating that had caused the patient such great psychological discomfort had decreased. In the words of the patient, the secretion of the sweat glands had already become normal a week after inserting the needles by the prolonged action method from an indifferent section to a point.

### Example 4. From one reflexogenic zone to another reflexogenic zone

Patient age 34, presented complaining of prolonged severe headaches. The patient had suffered from migraine since the age of 20. The attacks occurred spontaneously, and were not associated with external stimuli. Recently the pain had been alleviated by powerful non-narcotic analgesics, which however became ineffective in therapeutic doses. Objective findings: the patient's state was satisfactory, but the position of the head was constrained. Since active movements on turning the head intensified the pain, the patient held her head in a fixed attitude, slightly inclined forwardly, since in this position the pain was as it were held in check. On examining the cervical region of the spine movements in all direction were free of pain. A certain pastiness of the face was noted. On examination and palpation two regions behind the oracle were found to be very painful, both with a diameter equal to the size of the cushion of the little finger, and at a distance of 1 cm from one another. Not one acupuncture point was found during the topographical determination of the pain zones in this region. Adjacent were the points VB20 and VB12, although the epicentre of the pain in the form of two zones is located between these points. It was observed that some pain relief occurs on prolonged palpation of the painful zones.

At the appointment prolonged action acupuncture was carried out in the detected pain zones by insertion of a needle into one zone, with its end exiting from another zone, followed by securing the needle in the muscle layer using a fixing means. The needle was left in place for one month. At the next appointment one month later the patient said that the pain had become less within two hours after the last session. For the whole period the needle was in place the pain did not recur. The needle was left in place for a further month. At the next appointment the patient did not report any painful attacks. The patient's state was satisfactory and his sense of well-being was good. The needle was removed from the channel.

### Example 5. From a reflexogenic zone to a point

Patient age 65, presented complaining of pain in the region of the right shoulder blade, which became worse on moving the shoulder joint and if the head was held in one position for a long time. The pain was constant and irradiated into a specific region underneath the shoulder blade. On examination a forced position of the body was noted - the right shoulder was raised. On palpation it was found that the soft tissues on the affected side were painful and compacted. On deeper palpation a section of increased density the size of a bean seed was detected, which was extremely painful. At the same time the pain irradiated to the shoulder, and the neck was rather painful, which even caused the patient to cry out. The symptoms are caused by the development of a long-lasting spasm of part of the muscle fibres against a background of osteochondrosis of the cervical-thoracic region "trigger zone" (Shealy, D., Mortimer, J. Et Hagfor, N., 1970. - Dorsal column electroanalgesia. J. Neurosurg., 32, 560-564.). Conventional acupuncture at the trigger point and according to the recommended procedure in such situations did not provide any beneficial effect. Manual therapy of the patient was contraindicated on account of his age.

In the acupuncture section acupuncture was carried out by the prolonged action method in the projection of the trigger zone, the needle exiting at the local action point V45 located 3 cm away. The needle was fixed in situ by locking means, with the formation of a curved acusal tract, passing through the epidermal layer, epidermis membrane, papillary, reticular, epichondrial, subcutaneous-adipose and muscle layers, and was left in place for two weeks. Two weeks later at the next appointment the patient reported that he felt much better after three days. In his words: "I slept without any pain". On examination, no forced position of the body was found. Palpation revealed a residual painfulness in the trigger zone. The needle was left in place for a further two weeks. At the following appointment the pain had practically gone, but the needle was left for a further 10 weeks in order to consolidate the effect. At the next appointment two and a half months later the tissues in the acupuncture zone were soft and were not painful. The patient felt well. The pain did not recur.

### Example 6. From a reflexogenic zone to an indifferent zone

Patient age 23 presented complaining of persistent pain on the left side. The pain was a dull pain and became increasingly troublesome, since it did not disappear within a month. On examination of the internal organs no adverse pathology was detected. Objective findings: the patient's state was satisfactory. The stomach was soft and not painful. Physiological parameters in the normal range. On palpation of the region of the 6th rib and intercostal region a painful zone was detected along the mid-axillary line. The skin above this zone was unchanged. There was no sclerosis.

A centre of the above elective pain was found with the aid of blunt probe and a prolonged action needle was inserted in the centre, the other end of the needle exiting beyond the limits of the painful zone. The end of the needle was fixed in position by a removal locking means. The needle was left for three days in the subcutaneous-adipose layer. On examination the patient reported a "relief" of the pain. The needle was left in place for a further three days. At the next appointment the patient reported that she felt much better, and although the pain periodically recurred, on the whole the pain-free intervals became much longer. The next appointment was fixed for one month ahead. At this appointment the patient had no complaints. The painful symptoms had completely disappeared. The needle was removed from the channel.

### Example 7. The point itself at the limits of its boundary

A patient age 65 had suffered from bronchial asthma for 20 years. She took regular medication when the asthma became worse: in the spring and autumn. Between the periods of exacerbated asthma she took broncholytics. During the last two years the attacks of asthma were practically every day, mainly in the evening, throughout the whole year. Treatment at a health spa, treatment with drugs and conventional acupuncture did not produce any long lasting effect, merely providing temporary alleviation of the bronchial attacks. Objective findings: the patient had a normal diet and a normal constitution. The skin was of normal colour and clear. On auscultation of the lungs, the breathing was found to be hard and expiration was prolonged. The respiratory rate was 26 per minute, with expiratory dyspnea with the option of prescribing hormone treatment was raised.

Since it is generally accepted that hormone treatment is a treatment of "last resort", it was decided to carry out a course of prolonged acupuncture treatment on the whole set of points indicated for the treatment of the given nosology. For this, the following points on the auricle were chosen: AT13 (suprarenal), AT15 (neck and larynx), AT28 (hypophysas), AT51 (sympathetic), At31 (asthma), AT55 (shen-men). The following corporal points V11, V41, V42, V13, V43, V15, V17 were taken on the rear surface of the spine. For each separate point the topographical zone of this point was determined. A needle was inserted at the contour edge of the point and was passed through the whole point, the needle emerging from the other edge of the point, the ends being secured by a removable fixing means. Fine needles of length 2-5 mm were chosen for the acupuncture points, into the cartilage layer, and for the corporal points thicker ones were chosen, of length from 1 cm to 1.5 cm these needles were used to form a curved acusal tract, passing through the epidermal layer, epidermis membrane, papillary, reticular, epichondrial, subcutaneous-adipose and muscle layers. The needles were left in situ for 28 days. At the following appointment the patient reported a significant improvement in her well-being, and the frequency of the bronchial spasm attacks had decreased to 2 to 3 times a week. The needles were left in place for a further two months. The patient did not attend the next appointment, but reported on the phone that her sense of well-being was gradually improving and the attacks of breathlessness were already down to 1 to 2 times a week. She also said that this improvement allowed her to travel, which previously she had been unable to do on account of her asthma, and she had gone to visit her daughter in Sakhalin for three months. At the next appointment 10 months afterwards the patient reported that the attacks of breathlessness now occurred only once every 3-4 weeks, which were easily treated with broncholytics. Bearing in mind that her condition had stabilised and she was in a state of remission, the needles were removed from the acupuncture and corporal points.

### Example 8. Reflexogenic zone within the limits of its boundary

Patient, age 24, presented complaining of excess weight. On examination pronounced fat deposits of a gluteal-femoral type were detected. Objective findings - height 1.56 m, weight 90 kg. A session of prolonged acupuncture was carried out at points AT 17-18; a low calorie diet was prescribed The next appointment was fixed for one month ahead. At the next appointment the patient had gained 3 kg in weight. The patient complained of sharp taste sensations even on eating fresh, low-salt, unspiced food. The sensations lasted a long time in the form of taste experiences. This was hollowed by taste recollections and sometimes taste hallucinations.

In practice it has been observed that under the action on the points AT 17-18 (thirst, hunger) the appetite and feeling of thirst is blocked, although patients in fact often complain of sharp, clear taste experiences and taste memory.

It is necessary to employ prolonged action acupuncture on the glossopharyngeal nerve zone, which is located in the region of the salivary glands. There are no acupuncture points in this region, but it has been noted that with a braking action on this zone the taste sensations and salivation are reduced, since the sensitive fibres of this nerve receive taste stimuli, and the vegetative fibres regulate salivation. An action variant such as acupuncture of the zone of the base of the tragus by a prolonged action method with the formation of an acusal tract in the ligament layer, produces a pronounced therapeutic effect in some patients that is not achieved by conventional acupuncture at AT 17-18. The patient was treated by acupuncture of the given zone to act on the receptors 9 of the craniocerebal and glossopharyngeal pair of nerves. The next appointment was fixed for one month ahead. At the third appointment the patient's state was satisfactory. The patient no longer complained of the previous symptoms. The patient had lost 7 kg in weight. Thereafter, the patient lost weight in a stable manner, feeling well overall, and her weight dropped to normal body weight (63 kg) within six months.

The use of the described prolonged action acupuncture method provides an effective treatment, without the use of drugs, of a wide range of conditions and illnesses, and also increases the acupuncture effectiveness by prolonged action as a result of overcoming an adaptation effect, changing the intensity of the reflexogenic interaction, preventing complications, and also broadening the range of methods that can be used to treat various nosological conditions. The possibility of using relatively simple and inexpensive needle in the implementation of the method and with the minimum of side effects, enables the described method to be widely used in medical practice.

## Claims

1. An acupuncture needle, comprising a body (1) made of material able to be plastically flattened and at least one movable fixing means (2;3) provided with a through aperture, said fixing means (2;3) being installed on the body (1) of the needle and capable of sliding, and its securement in a given position is effectable by flattening plastic deformation (4) of the body (1) of the needle at the fixing means distal side_so as the width of the flattened area exceeds the diameter of the said through aperture.

2. An acupuncture needle according to claim 1, wherein the fixing means (2, 3) are installed on the ends of the body (1) and at least one which is movable being capable of sliding along the body (1).

3. An acupuncture needle according to claim 2, wherein the non movable fixing means (2) is secured in a given position by flattening plastic deformation (4,5)of said body (1) at both ends of said fixing means.

4. The needle as claimed in any of claims 1 to 3, wherein the body (1) of the needle is made rigid in the form of a rod.

5. The needle as claimed in any of claims 1 to 3, wherein the body (19) of the needle is made flexible in the form of a string.

6. The needle as claimed in any of claims 1 to 5, wherein the body (19) of the needle is made plastic metal alloy.

## Patentansprüche

1. Akupunkturnadel, umfassend einen Körper (1), der aus einem Material gebildet ist, das in der Lage ist, plastisch abgeflacht zu werden, und zumindest ein bewegliches Fixiermittel (2; 3), das mit einer Durchgangsöffnung versehen ist, wobei das Fixiermittel (2; 3) auf dem Körper (1) der Nadel installiert und in der Lage ist, zu gleiten, und wobei dessen Sicherung in einer bestimmten Position durch eine plastische Abflachungsverformung (4) des Körpers (1) der Nadel auf einer distalen Seite des Fixiermittels bewirkbar ist, so dass die Breite des abgeflachten Bereichs den Durchmesser der Durchgangsöffnung überschreitet.

2. Akupunkturnadel nach Anspruch 1, wobei die Fixiermittel (2, 3) auf den Enden des Körpers (1) installiert sind und zumindest eines davon beweglich und in der Lage ist, entlang des Körpers (1) zu gleiten.

3. Akupunkturnadel nach Anspruch 2, wobei das unbewegliche Fixiermittel (2) durch eine plastische Abflachungsverformung (4, 5) des Körpers (1) an beiden Enden des Fixiermittels in einer bestimmten Position gesichert wird.

4. Nadel nach einem der Ansprüche 1 bis 3, wobei der Körper (1) der Nadel in Form einer Stange steif ausgebildet ist.

5. Nadel nach einem der Ansprüche 1 bis 3, wobei der Körper (19) der Nadel in Form einer Schnur flexibel ausgebildet ist.

6. Nadel nach einem der Ansprüche 1 bis 5, wobei der Körper (19) der Nadel aus einer Kunststoff-Metall-Legierung gebildet ist.

## Revendications

1. Aiguille d'acupuncture comprenant un corps (1) réalisé avec un matériau pouvant être plastiquement aplati et au moins un moyen de fixation mobile (2; 3) prévu avec une ouverture débouchante, ledit moyen de fixation (2; 3) étant installé sur le corps (1) de l'aiguille et pouvant coulisser, et sa fixation dans une position donnée peut être effectuée en par déformation plastique d'aplatissement (4) du corps (1) de l'aiguille au niveau du côté distal des moyens de fixation de sorte que la largeur de la zone aplatie dépasse le diamètre de ladite ouverture débouchante.

2. Aiguille d'acupuncture selon la revendication 1, dans laquelle les moyens de fixation (2, 3) sont installés sur les extrémités du corps (1) et dont au moins un est mobile en pouvant coulisser le long du corps (1).

3. Aiguille d'acupuncture selon la revendication 2, dans laquelle le moyen de fixation non mobile (2) est fixé dans une position donnée par déformation plastique d'aplatissement (4, 5) dudit corps (1) aux deux extrémités dudit moyen de fixation.

4. Aiguille selon l'une quelconque des revendications 1 à 3, dans laquelle le corps (1) de l'aiguille est rendu rigide sous la forme d'une tige.

5. Aiguille selon l'une quelconque des revendications 1 à 3, dans laquelle le corps (19) de l'aiguille est rendu flexible sous la forme d'un fil.

6. Aiguille selon l'une quelconque des revendications 1 à 5, dans laquelle le corps (19) de l'aiguille est réalisé avec un alliage de métal plastique.
